(19) Europäisches Patentamt
European Patent Office
Office européen des brevets

(11) EP 2 563 414 B1

(12) **FASCICULE DE BREVET EUROPEEN**

(45) Date de publication et mention de la délivrance du brevet:
**19.03.2014 Bulletin 2014/12**

(21) Numéro de dépôt: **11723515.0**

(22) Date de dépôt: **27.04.2011**

(51) Int Cl.:
***A61L 15/22*** *(2006.01)* ***A61L 15/42*** *(2006.01)*
***A61L 15/58*** *(2006.01)*

(86) Numéro de dépôt international:
**PCT/FR2011/050955**

(87) Numéro de publication internationale:
**WO 2011/135255 (03.11.2011 Gazette 2011/44)**

(54) **PANSEMENT AMPOULE COMPRENANT AU MOINS DEUX RESINES TACKIFIANTES**

BRANDBLASENVERBAND MIT MINDESTENS ZWEI KLEBRIGEN HARZEN

BLISTER DRESSING INCLUDING AT LEAST TWO TACKIFYING RESINS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.04.2010 FR 1053263**

(43) Date de publication de la demande:
**06.03.2013 Bulletin 2013/10**

(73) Titulaire: **Laboratoires Urgo**
**21300 Chenove (FR)**

(72) Inventeur: **AUGUSTE, Stéphane**
**F-21490 Varois et Chaignot (FR)**

(74) Mandataire: **Hubert, Philippe et al**
**Cabinet Beau de Loménie**
**158, rue de l'Université**
**75340 Paris Cedex 07 (FR)**

(56) Documents cités:
**WO-A1-99/27014** **WO-A1-2009/146227**
**FR-A1- 2 753 380** **FR-A1- 2 775 903**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 563 414 B1

## Description

La présente invention a pour objet un nouveau pansement comprenant une masse hydrocolloïde adhésive destiné au traitement des plaies telles que les plaies exudatives, les brûlures, les lésions dermo-épidermiques superficielles, profondes, chroniques ou aiguës et en particulier au traitement de l'ampoule. Les pansements selon l'invention présentent une tenue dans le temps améliorée.

Arrière-plan technologique

Les pansements comprenant des hydrocolloïdes sont connus depuis plus de 20 ans. Ils sont constitués d'un support sur lequel est déposée une masse adhésive comprenant des hydrocolloïdes. On peut citer, à titre d'exemples, les produits commercialisés sous les dénominations Algoplaque® par les Laboratoires URGO et Comfeel® par la société Coloplast. Des pansements comprenant une masse adhésive comprenant des hydrocolloïdes, spécifiquement destinés au traitement de l'ampoule, sont également connus et commercialisés par exemple sous les dénominations Urgo Traitement Ampoules® par les Laboratoires URGO et Compeed® par la société Johnson & Johnson.

Pour permettre une bonne absorption des exsudats de la plaie, ces pansements contiennent des quantités relativement importantes (de l'ordre de 20 à 50 % en masse) d'hydrocolloïdes. De manière préférentielle, ces pansements sont conçus pour tenir en place sans l'aide d'une bande adhésive complémentaire, en adhérant directement à la peau.

La masse adhésive de ces pansements connus est habituellement constituée d'une phase continue hydrophobe, généralement à base d'élastomères, dans laquelle est dispersée une phase discontinue de particules d'hydrocolloïdes destinées à absorber les exsudats de la plaie.

L'absorption des exsudats par les hydrocolloïdes provoque la gélification de la masse adhésive, ce qui permet de retirer sans douleur le pansement de la plaie après son utilisation.

D'une façon générale, ces masses adhésives comprennent, outre la matrice élastomérique contenant des particules hydrocolloïdes, un (ou plusieurs) composé(s) destiné(s) à conférer des propriétés d'adhérence à ladite masse connu(s) sous le nom de « tackifiant(s) ».

Pour assurer le maintien dans le temps de leur capacité d'absorption et de leur cohésion lors du retrait, ces pansements possèdent un pouvoir adhésif initial important. Ceci est encore plus vrai pour les pansements destinés au traitement des ampoules qui doivent être positionnés dans des zones courbes ou difficiles d'accès et qui sont soumis à de fortes contraintes mécaniques lors de leur utilisation.

Des pansements comprenant des particules d'hydrocolloïdes dispersées dans une matrice élastomérique, sont par exemple décrits dans les documents FR 2 495 473, FR 2 775 903, EP 0 927 051, EP 1 165 717.

Le document EP 0 264 299 décrit un pansement présentant ce type de composition dans la couche de masse adhésive, étant par ailleurs précisé que le coussinet d'étanchéité, au moins sur sa périphérie, est conformé en biseau de manière telle que son épaisseur près de son rebord ne dépasse pas environ un quart de son épaisseur maximale. WO 92/05755 décrit également un pansement comprenant des particules d'hydrocolloïdes dispersées dans une matrice élastomérique, caractérisé en ce qu'il présente un large rebord périphérique d'une épaisseur inférieur à 0,5 mm et qui s'étend sur au moins 10 mm. EP 1 020 198 décrit un dispositif semblable à celui du document WO 92/05755, la partie rebord périphérique, d'épaisseur 0,15 à 0,20 mm, s'étendant cette fois-ci jusqu'à une distance de 3,0 mm de la partie centrale épaisse, d'épaisseur 0,5 mm.

Problème(s) à résoudre

Un pansement idéal doit répondre si possible à un certain nombre d'exigences lors de l'utilisation par le patient présentant une plaie, dont certaines sont par essence difficiles à satisfaire simultanément. Une trop forte adhésion, bien que permettant d'assurer l'application du pansement, peut avoir comme conséquence un reliquat indésirable de masse adhésive sur la peau au moment de son retrait. Une trop forte adhésion peut également entraîner un retrait douloureux du pansement et/ou provoquer une desquamation superficielle de la peau. Une viscosité trop élevée de la masse adhésive peut empêcher cette dernière de suivre les déplacements du corps du porteur, mais une masse adhésive trop fluide finira par déborder de son cadre. Si un pansement comprenant une masse adhésive trop fluide est soumis à une force perpendiculaire à sa surface, cela aura pour conséquence de faire fluer la masse au niveau des bords périphériques. Si un tel pansement est soumis à une force dans le plan du pansement, cela aura pour conséquence d'excentrer le pansement de son site de pose. Le support va glisser et pourra dans certains cas provoquer un délaminage de la masse et du support. On vise en effet à satisfaire autant que possible les besoins pendant les phases distinctes de la vie du pansement, que l'on peut définir comme l'accostage, le port et le retrait.

Dans le cadre de la présente invention, des recherches ont été entreprises afin de développer des masses adhésives octroyant à des pansements les contenant une meilleure tenue sur la peau. Les mouvements naturels du corps humain en eux-mêmes font subir des forces mécaniques à des pansements, lesquels sont sujets également à une température au-delà de la température ambiante ainsi qu'à des apports d'eau, de graisses naturelles, sans mentionner les exsudats des plaies qu'ils sont censés isoler de l'extérieur. L'action du corps humain sur le pansement se réalise souvent à l'intérieur de vêtements ou d'autres accessoires tels que des chaussures, ce qui augmente notamment les forces mécaniques. La tendance des pansements à être détachés du corps humain augmente naturellement avec le degré d'activité du porteur, les sportifs connaissant un taux élevé de perte des pansements. Tout en assurant une bonne tenue sur la peau, il est souhaitable de disposer d'un bon pouvoir adhésif initial avec un « tack » suffisant pour ancrer de façon sure le pansement sur la peau lors de sa pose initiale. De préférence, ce « tack » sera stable dans le temps, afin d'assurer une durée de conservation envisageable la plus longue possible après fabrication. De façon générale, on désire disposer de pansements présentant une certaine résistance à l'eau (permettant ainsi au porteur de se laver au moins au moyen d'une douche sans perte du pansement), qui procurent au porteur une sensation de protection de la peau endommagée et qui ne transfèrent pas de quantité significative de masse adhésive sur la peau. Il est également souhaitable que la masse adhésive soit stable dans le temps, de façon à ce qu'après des mois, voire des années de stockage, ses propriétés ne sont pas altérées de façon significative.

Résumé de Invention

Il a été découvert de façon surprenante, et ceci constitue le fondement de la présente invention, qu'un pansement comprenant des particules d'hydrocolloïde(s) dispersées dans une matrice élastomérique, dans lequel la couche de masse adhésive hydrocolloïde comprend deux résines tackifiantes d'hydrocarbure hydrogéné dont les points de ramollissement diffèrent d'au moins 10°C et d'au plus 40°C, présente une tenue améliorée, telle que constatée dans des tests réalisés sur des porteurs volontaires, sans compromettre les propriétés recherchées comprenant notamment la résistance à l'eau, la sensation de protection et l'absence de transfert significatif de masse adhésive sur la peau, une bonne adhésivité initiale et une bonne stabilité de masse adhésive dans le temps.

La présente invention se rapporte donc à un pansement comprenant une masse adhésive hydrocolloïde comprenant :

- une matrice élastomérique hydrophobe comprenant des copolymères séquencés poly(styrène-oléfine-styrène),
- des particules d'hydrocolloïde(s) dispersées dans ladite matrice élastomérique,
- au moins deux résines tackifiantes d'hydrocarbure hydrogéné, la différence entre les températures de ramollissement d'une première résine tackifiante d'hydrocarbure hydrogéné et d'une deuxième résine tackifiante d'hydrocarbure hydrogéné comprises dans la masse adhésive hydrocolloïde étant d'au moins 10°C et d'au plus 40°C.

Brève description des Figures

La Figure 1 représente un profil d'épaisseur d'un pansement à bords amincis, un mode de réalisation possible de la présente invention.

La Figure 2 représente un dispositif de mesure de fluage de masses adhésives hydrocolloïdes tel qu'utilisé dans l'étude en laboratoire de telles masses dans le cadre de la présent invention.

La Figure 3 présente des résultats obtenus en matière de décollage de pansements ampoule de forme ovale sur porteurs sportifs volontaires, dans une étude comparative avec le produit commercial Compeed®.

La Figure 4 représente un dispositif de mesure de tack d'un matériau adhésif à l'aide d'une sonde cylindrique.

Description détaillée de l'invention

Les pansements selon la présente invention comprennent une couche de masse adhésive hydrocolloïde comprenant les éléments essentiels que sont la matrice hydrophobe élastomérique, les particules d'hydrocolloïde(s) ainsi que la combinaison d'au moins deux résines tackifiantes d'hydrocarbure hydrogéné comme indiqué plus haut. Les pansements selon la présente invention peuvent en outre présenter, sur la face de la masse adhésive hydrocolloïde non destinée à être au contact de la peau du patient, un support. Les pansements selon la présente invention peuvent également présenter, sur la face de la masse adhésive hydrocolloïde destinée à être au contact de la peau du patient, un film de protection. Selon un mode de réalisation de la présente invention, le pansement présente une forme à bords amincis. Le pansement vu de dessus peut revêtir une forme carrée ou rectangulaire. De même sa taille peut être adaptée librement en fonction de la surface de la partie à traiter ou à protéger. Par exemple, un pansement destiné au traitement de l'ampoule peut se présenter sous une forme rectangulaire d'environ 7 cm de longueur et d'environ 4 cm de largeur tandis qu'un pansement destiné au traitement de l'ulcère peut convenablement se présenter sous une forme carrée de 10 cm de coté. Des formes autres que carrée ou rectangulaire sont également envisagées pour des pansements selon l'invention, par exemple des formes circulaires, ovales ou présentant l'apparence d'un haricot.

La matrice élastomérique de la couche masse adhésive hydrocolloïde comprend des copolymères séquences poly(styrène-oléfine-styrène). De préférence, la matrice élastomérique comprend un (ou plusieurs) élastomère(s) appartenant à la famille des copolymères triblocs poly(styrène-isoprène-styrène) (en abrégé : poly(SIS)) et les mélanges de copolymères triblocs poly(SIS) et de copolymères diblocs poly(styrène-isoprène), et en particulier les poly(SIS) ayant une teneur en styrène comprise entre 14 et 52 % et de préférence entre 14 et 30 % en poids rapporté au poids dudit poly(SIS).

De tels produits bien connus de l'homme de l'art sont par exemple commercialisés par la société Kraton sous la dénomination KRATON®D ou par la société Dexco Polymers LP sous la dénomination VECTOR®.

Parmi les copolymères triblocs poly(SIS) préférés, on peut citer en particulier les produits commercialisés sous les dénominations KRATON®D-1111K, KRATON®D-1111CS, KRATON®D-1107 ou KRATON®1161, VECTOR®4114 et VECTOR®4113.

Des copolymères triblocs poly(styrène-butadiène-styrène) peuvent également être utilisés dans le cadre de l'invention.

Parmi ces copolymères poly(styrène-butadiène-styrène), on peut citer en particulier le produit commercialisé sous la dénomination KRATON®D-1102 par la société Kraton.

De préférence, les élastomères formant la matrice élastomérique seront présents, au sein de la masse adhésive des pansements selon l'invention en une quantité de 10 à 30 % en poids, et de préférence de 15 à 25 % en poids, du poids total de la masse adhésive hydrocolldide.

D'une façon générale, la matrice élastomérique précitée incorpore un (ou plusieurs) hydrocolloïde(s).

Par « hydrocolloïde» on entend désigner ici tout composé habituellement utilisé par l'homme de l'art pour son aptitude à absorber les liquides hydrophiles tels que l'eau, le sérum physiologique ou les exsudats d'une plaie.

A titre d'exemple d'hydrocolloïde susceptible d'être utilisé dans le cadre de l'invention, on peut citer la pectine, les alginates, les gommes végétales naturelles comme en particulier la gomme de Karaya, les dérivés de cellulose comme en particulier les carboxyméthylcelluloses et leurs sels de métal alcalin, notamment de sodium ou de calcium, ainsi que les polymères synthétiques à base de sels de l'acide acrylique, connus sous l'appellation "superabsorbants", comme par en particulier les produits commercialisés par la société BASF sous la dénomination LUQUASORB® 1003 ou par la société CIBA Specialty chemicals sous la dénomination SALCARE® SC91. Bien entendu, des mélanges de ces produits peuvent être utilisés à titre d'hydrocolloïdes.

Les hydrocolloïdes préférés dans le cadre de la présente invention sont les sels de métal alcalin de la carboxyméthylcellulose, et en particulier la carboxyméthylcellulose de sodium.

La quantité d'hydrocolloïde(s) incorporée dans la matrice élastomérique sera adaptée en fonction du niveau d'absorption souhaité. D'une façon générale, la quantité d'hydrocolloïde(s) pourra être de l'ordre de 2 à 50 % en poids, rapportée au poids total de la masse adhésive hydrocolloïde.

Dans le cadre de la présente invention, on utilisera de préférence une quantité d'hydrocolloïde(s) comprise entre 20 et 50 % en poids rapportée au poids total de la masse adhésive hydrocolloïde.

La matrice élastomérique contenant des particules hydrocolloïdes est rendue adhésive par l'ajout de produits dits « tackifiants ». Des produits tackifiants susceptibles d'être utilisés dans le cadre de la présente invention sont les résines tackifiantes d'hydrocarbure(s) hydrogéné(s). De tels produits sont commercialisés par exemple par la société ARAKAWA sous la dénomination ARKON®. Ces produits sont vendus par grades indiquant la température de ramollissement. La température du point de ramollissement de l'ARKON® P 90 est de 90°C, celle de l'ARKON® P 125 est de 125°C et celle de l'ARKON® P 140 est de 140°C. Les résines ARKON® P 100 et ARKON® M 100 sont préparés par l'hydrogénation de résines d'hydrocarbures aromatiques obtenues par polymérisation cationique de la fraction C9 avec des points d'ébullition compris entre 140 et 280°C, cette fraction étant substantiellement exempte de fraction en C5. Dans le cas de la résine ARKON® P 100, le degré d'hydrogénation est considéré comme total, tous les noyaux aromatiques étant hydrogénés. Dans le cas de la résine ARKON® M 100, l'hydrogénation est incomplète, le degré d'hydrogénation étant de 50 à 80%. Dans la mesure où les produits de départ avant hydrogénation de résines tackifiantes telles que celle de la série ARKON® sont aromatiques, de telles résines sont également couramment appelées « résines tackifiantes aromatiques hydrogénées », bien que le produit final peut ne plus contenir de noyau aromatique.

Dans la présente invention, au moins deux résines tackifiantes d'hydrocarbure(s) hydrogéné(s) doivent être présentes, dont les points de ramollissement diffèrent d'au moins 10°C et d'au plus 40°C, de préférence d'au moins 20°C et d'au plus 40°C, encore plus préférentiellement d'au moins 30°C et d'au plus 40°C. Il s'agit de préférence de deux résines tackifiantes dans la classe de résines aromatiques hydrogénées telle que détaillées plus haut. De manière préférentielle, le point de ramollissement de la résine tackifiante dont le point de ramollissement est le plus bas sera d'au moins 65°C, de préférence d'au moins 80°C. De manière préférentielle, le point de ramollissement de la résine tackifiante dont le point de ramollissement est le plus haut sera d'au plus 145°C, de préférence d'au plus 130°C.

Il a été observé que l'utilisation d'une seule résine tackifiante aromatique hydrogénée peut réduire la capacité à présenter un bon pouvoir adhésif initial avec un « tack » suffisant pour ancrer de façon sure le pansement sur la peau lors de sa pose initiale ainsi que de conserver ce pouvoir adhésif initial lors de la pose pendant une longue durée de conservation du pansement. L'utilisation de deux résines tackifiantes aromatiques hydrogénées permet de réconcilier au mieux l'ensemble de propriétés recherchées.

En plus des résines tackifiantes d'hydrocarbure(s) (aromatiques) hydrogénée(s), la masse adhésive hydrocolloïde peut éventuellement contenir d'autres familles de résines tackifiantes, par exemple les produits obtenus par polymérisation de monomères aliphatiques en C5 (telles que la série WINGTACK®), des polyterpènes ou des résines de colophanes. Parmi les résines tackifiantes utilisées dans le domaine des adhésifs, on distingue généralement entre, d'une part, des résines d'hydrocarbures (synthétiques) et, d'autre part, des résines d'origine naturelle (bien que ces dernières puissent être modifiées chimiquement). Parmi les résines d'hydrocarbures (synthétiques), on retrouve notamment:

- les résines de type aromatique / C9 (précédemment décrites en détail et applicables dans des pansements selon l'invention). Ces résines sont généralement obtenues par l'hydrogénation de résines obtenues par polymérisation de monomères aromatiques telle que le styrène, l'$\alpha$-méthylstyrène, le vinyltoluène, l'indène, et le méthylindène ;
- les résines de type aliphatique / C5, généralement obtenues par la polymérisation de monomères tels que l'isoamylène, le cyclopentène et le piperylène ;
- les résines à base d'indène et de coumarone ; et
- les résines DCPD à base de dimères du cyclopentadiène.

Parmi les résines d'origine naturelle, on retrouve notamment :

- les résines de type (poly)terpène, obtenues par polymérisation de monomères terpènes (tels que le pinène, le camphène ou le limonène), éventuellement avec un comonomère synthétique tel que le styrène, et/ou modifiées par réaction avec un phénol ; et
- les résines de colophanes. Par « colophane » (également appelé « rosine »), on fait généralement référence à une substance obtenue à partir des arbres résineux tels que des pins. La résine obtenue est majoritairement composée d'acides organiques de la famille des diterpènes. Divers composés diterpéniques peuvent être présents, le squelette majoritaire étant celui de l'acide abiétique. Il est possible d'hydrogéner des acides résiniques tels que l'acide abiétique et ses congénères, qui à l'état naturel présentent des liaisons C=C. En outre, le groupe $-CO_2H$ dans les acides résiniques peut être estérifié, et les esters avec des polyols (glycols, pentaérythritol etc.) sont bien connus.

De préférence, dans les pansements de la présente invention, des résines autres que des résines tackifiantes d'hydrocarbure(s) (aromatiques) hydrogéné(s) ne constituent qu'au plus 5% en poids par rapport au poids total de la masse adhésive hydrocolloïde, et plus préférentiellement la masse adhésive hydrocolloïde est substantiellement exempte de résines tackifiantes autres que des résines tackifiantes d'hydrocarbure(s) hydrogéné(s).

De préférence, le(s) produit(s) tackifiant(s) représentera (représenteront) de 10 à 40 % en poids, du poids total de la masse adhésive hydrocolloïde, préférentiellement de 25 à 35%. De préférence, chacune des deux résines tackifiantes d'hydrocarbure(s) hydrogéné(s), dont les points de ramollissement diffèrent d'au moins 10°C et d'au plus 40°C, représentera au moins 5%, de préférence au moins 10% en poids du poids total de la masse adhésive hydrocolloïde.

Divers composés additionnels pourront être ajoutés à la matrice élastomérique contenant les composés tackifiants et hydrocolloïdes précités pour obtenir des masses adhésives hydrocolloïdes qui présentent des propriétés d'élasticité, d'adhésion, de stabilité dans le temps et de cohésion optimisées.

De tels composés sont par exemple des stabilisants comme en particulier des antioxydants, des plastifiants comme en particulier les huiles plastifiantes.

Par « stabilisant » on entend désigner ici tout composé apte à assurer la stabilité vis-à-vis de l'oxygène (antioxydant), la chaleur, l'ozone et les rayonnements ultra violets des composés utilisés dans la formulation des masses hydrocolloïdes adhésives, en particulier des résines tackifiantes et des copolymères séquencés. Ces composés stabilisants sont bien connus et pourront être utilisés seuls ou en mélange.

Parmi les composés antioxydants susceptibles d'être utilisés selon l'invention, on peut citer les antioxydants phénoliques, comme par exemple les produits commercialisés par la société CIBA-GEIGY sous les dénominations IRGANOX®1010, IRGANOX®565 et IRGANOX® 1076 ainsi que les antioxydants soufrés, comme par exemple le dibutyldithiocarbamate de zinc commercialisé par la société FLEXSYS sous la dénomination PERKACIT®ZDBC.

Ces composés peuvent être utilisés seuls ou en mélange, de préférence dans une proportion de 0 à 2 % en poids, et plus particulièrement de 0,1 à 0,6 % en poids, rapportée au poids total de la masse adhésive hydrocolloïde.

Dans le cadre de la présente invention, on utilisera de façon préférée l'association de l'IRGANOX®1010 et du PERKACIT®ZDBC.

Parmi les composés plastifiants susceptibles d'être utilisés selon l'invention, on peut citer les plastifiants habituellement utilisés par l'homme de l'art pour la préparation de masses adhésives hydrocolloïdes, les huiles plastifiantes ou encore des dérivés de phtalate tels que le dioctylphtalate, ou bien des adipates.

L'utilisation des huiles plastifiantes est particulièrement préférée dans le cadre de la présente invention.

Par « huile plastifiante » on entend désigner ici les huiles minérales ou végétales couramment employées par l'homme de l'art pour plastifier les copolymères séquences du type styrène-oléfine-styrène utilisés dans la composition des masses adhésives hydrocolloïdes.

Ces huiles minérales sont généralement constituées de mélanges dans des proportions variables de composés de nature paraffinique, naphténique ou aromatique.

Parmi les huiles plastifiantes susceptibles d'être utilisées selon l'invention, on peut citer les produits commercialisés par la société SHELL sous les dénominations ONDINA® et RISELLA® qui sont constitués de mélanges à base de composés naphténiques et paraffiniques ou sous la dénomination CATENEX® qui sont constitués de mélanges à base de composés naphténiques, aromatiques et paraffiniques, les produits commercialisés par Croda sous la dénomination Crodamol DOA ou commercialisés par Eigenmann & Veronelli sous la dénomination commerciale Lincol DOA-C qui sont du diethylhexyl adipate.

Dans le cadre de la présente invention, on utilisera de façon préférentielle le diethylhexyl adipate commercialisé sous la dénomination Crodamol DOA par Croda.

Ces composés plastifiants peuvent être utilisés seuls ou en mélange, de préférence dans une proportion de 5 à 20 % en poids, et plus particulièrement de 7 à 15 % en poids, rapportée au poids total de la masse adhésive hydrocolloïde.

La masse adhésive hydrocolloïde des pansements selon l'invention peut encore comprendre un ou plusieurs composé(s) tensioactif(s) en une quantité inférieure ou égale à 10 % en poids, de préférence inférieure ou égale à 5 % en poids, rapportée au poids total de la masse adhésive hydrocolloïde.

Un composé tensioactif préféré dans le cadre de la présente invention est le composé commercialisé sous la dénomination AcResin®.

Un autre composé tensioactif préféré dans le cadre de la présente invention est le polysorbate 80, comme par exemple le produit commercialisé par la société SEPPIC sous la dénomination MONTANOX® 80.

L'épaisseur de la couche de masse adhésive hydrocolloïde des pansements selon l'invention est variable, selon l'utilisation envisagée. De préférence, l'épaisseur de la couche de masse adhésive hydrocolloïde variera entre au moins 0,3 mm et au plus 2,0 mm. Les épaisseurs élevées, de 1,2 mm et au-delà, par exemple entre 1,2 mm et 1,5 mm, seront préférées pour des plaies chroniques et dans d'autres situations dans lesquelles le volume des exsudats attendus est important. Bien que l'épaisseur de la couche de masse adhésive hydrocolloïde puisse être substantiellement la même sur toute la surface du pansement selon un mode de réalisation de la présente invention, selon un autre mode de réalisation, l'épaisseur est réduite aux bords.

Selon un mode préférentiel de réalisation de l'invention, le pansement est préparé de telle sorte que le profil en hauteur du pansement en allant du centre géométrique du pansement vers la périphérie du pansement soit constitué de trois sections, comme cela est représenté à la Figure 1 :

- une partie centrale comprenant le centre géométrique du pansement dans laquelle la hauteur E du profil (l'épaisseur maximale du pansement sur sa partie centrale) est substantiellement constante ;
- une partie périphérique d'épaisseur e substantiellement constante ;
- une zone de transition dans laquelle la courbure de raccordement liant les parties périphérique et centrale peut être représentée par une fonction parabolique de formule générale (1) :

$$y = a.x^2 + b.x + c,$$

avec a < 0

dans laquelle x représente la distance horizontale sur le profil (selon la représentation donnée à la Figure 1, x=0 au début de la zone de transition), et y représente la hauteur (épaisseur du pansement) dans la zone de transition, étant précisé que :

$$0{,}0010\ mm^{-1} \leq -a \leq 0{,}0036\ mm^{-1},$$

et

$$0{,}060 \leq b \leq 0{,}130.$$

Une surface de pansement pourrait naturellement présenter des aspérités mais les valeurs à retenir seront celles obtenues de la fonction parabolique qui présente le moins de déviation possible de la courbe observée en surface (par exemple par étude sous microscope). Bien entendu, la valeur « a » dans la fonction parabolique détermine l'effet du terme en $X^2$ et donc le degré de courbure.

Sans vouloir être lié par une interprétation théorique particulière, la Demanderesse considère que le fait de disposer d'un profil comme indiqué plus haut, sans rebord suivi de changement d'épaisseur très abrupte (comme décrit dans WO 92/05755 ou EP 1 020 198) ni bord biseauté (comme dans EP 0 264 299), présente des avantages permettant de réconcilier résistance au décollement du pansement avec bonne absorption des exsudats. En effet, un mauvais accostage peut dès le début provoquer de petites plissures du pansement, notamment au niveau des bords périphériques. Ces petites plissures permettront l'entrée d'air/eau/salissure à l'origine d'un décollement précoce. Aussi, de petites plissures facilitent l'accroche avec les chaussettes/chaussures/lanières qui provoquent le décollement du pansement. Si le pansement ne présente pas de petites plissures à la pose, les frottements des chaussettes/chaussures/lanières pourront être à l'origine du décollement. En fonction de la courbure, la surface en contact avec la chaussette/chaussure/lanière ne sera pas la même et la chaussette/chaussure/lanière glissera plus ou moins facilement sur cette surface. Selon le profil parabolique de cet aspect de la présente invention, le passage entre l'épaisseur du rebord périphérique et l'épaisseur maximale se fait de manière progressive. Il n'y a pas de passage abrupt et cela permet un meilleur glissement. Un passage progressif avec un bord biseauté (tel que décrit dans le brevet EP 0 264 299) nécessite une pente très faible, ce qui engendre une épaisseur moins importante de la masse sur une surface plus importante et par voie de conséquence une absorption plus faible des exsudats (car ce sont les particules hydrocolloïde(s) contenues dans la masse qui permettent une bonne absorption des exsudats).

Le pansement vu de dessus peut revêtir une forme carrée ou rectangulaire. De même sa taille peut être adaptée librement en fonction de la surface de la partie à traiter ou à protéger. Par exemple, un pansement destiné au traitement de l'ampoule peut se présenter sous une forme rectangulaire d'environ 7 cm de longueur et d'environ 4 cm de largeur tandis qu'un pansement destiné au traitement de l'ulcère peut convenablement se présenter sous une forme carrée de 10 cm de coté. Des formes autres que carrée ou rectangulaire sont également envisagées pour des pansements selon l'invention, par exemple des formes circulaires, ovales ou présentant l'apparence d'un haricot.

Dans le cas d'une forme rectangulaire ou ovale, on distingue clairement deux axes distincts traversant le centre géométrique, d'une part un axe transversal dont la longueur d'un bord périphérique à l'autre bord en passant par le centre est la plus courte possible (l'axe transversal), et d'autre part l'axe longitudinal, perpendiculaire a l'axe transversal et plus long que ce dernier.

Selon un mode préférentiel de la présente invention, le pansement, notamment de type ovale ou rectangulaire, comporte des axes longitudinal et transversal distincts passant par le centre géométrique du pansement, dans lequel :

- sur l'axe longitudinal, 0,0010 $mm^{-1} \leq -a \leq$ 0,0016 $mm^{-1}$, et 0,060 $\leq b \leq$ 0,0920, et
- sur l'axe transversal, 0,0020 $mm^{-1} \leq - a \leq$ 0,0036 $mm^{-1}$, et 0,080 $\leq b \leq$ 0,130.

En sens longitudinal donc, dans ce mode de réalisation préférentiel, la courbure de raccordement est plus faible qu'en sens transversal.

De préférence, dans les pansements à bords amincis dans ce mode de réalisation de la présente invention, la hauteur E dans la partie centrale du pansement d'épaisseur substantiellement constante est d'au moins 0,80 mm et d'au plus 1,2 mm, et la hauteur e dans la partie périphérique du pansement d'épaisseur substantiellement constante est d'au moins 0,26 mm et d'au plus 0,40 mm. Selon un mode préférentiel, l'épaisseur du pansement, dans sa partie la plus épaisse, comprenant la masse adhésive et un film de protection et une couche support, est d'environ 0,85 mm.

Des pansements présentant des profils géométriques tels que définis par le profil parabolique ci-dessus peuvent notamment être obtenus par la mise en oeuvre du procédé de la demande de brevet WO 2010/004222 de la Demanderesse, un cylindre comportant l'empreinte des formes géométriques souhaitées étant utilisé pour préparer de tels pansements à bord amincis.

Les pansements de l'invention peuvent être de différents types mais comprendront de préférence un support.

D'une façon générale, le choix du support sera réalisé en fonction des propriétés requises (étanchéité, élasticité, etc.) dans l'application recherchée.

Ainsi, le pansement selon l'invention peut comprendre un support tel qu'un film formé d'une ou plusieurs couches et d'une épaisseur variable de 5 à 150 $\mu$m ; un non-tissé ou encore une mousse ayant une épaisseur de 10 à 500 $\mu$m sur lequel la masse adhésive hydrocolloïde a été enduite, de façon continue ou non continue. Le plus souvent, le support présentera une épaisseur global de 10 à 100 $\mu$m, de préférence de 20 à 50 $\mu$m.

Ces supports à base de matériaux synthétiques ou naturels sont bien connus de l'homme de l'art.

Parmi les supports en forme de mousse utilisables dans le cadre de l'invention, on peut ainsi citer les mousses en polyéthylène, en polyuréthane, en PVC par exemple.

Parmi les supports non-tissés utilisables dans le cadre de l'invention, on peut citer les non-tissés en polypropylène, polyéthylène, polyuréthane, polyamide, ou polyester par exemple.

Dans le cadre de la présente invention, on préférera utiliser des supports en forme de films, et notamment des films en polyuréthanne comme par exemple les films commercialisés par la société Smith et Nephew sous la référence LASSO® réalisés à partir du polyuréthanne commercialisé par la société BF GOODRICH sous la dénomination ESTANE® ; des films en polyéthylène à basse densité comme par exemple les films commercialisés par la société SOPAL ; des films à base de copolymère thermoplastique de polyéther-polyester comme par exemple les produits commercialisés par la société DUPONT DE NEMOURS sous la dénomination Hytrel® ; ou encore des films complexes associant un film de polyuréthanne et un non-tissé.

Selon une variante de réalisation de l'invention, le pansement peut comporter une couche absorbante disposée entre le support et la masse adhésive hydrocolloïde. Cette couche absorbante peut être constituée de tout type de matériau absorbant tel que par exemple une mousse (comme en particulier une mousse polyuréthane), un non-tissé, une couche de polymère super absorbant, ou une combinaison de ces matériaux. Une couche absorbante de ce type, qui peut permettre d'absorber de grands volume d'exsudats, peut présenter une épaisseur allant de 50 $\mu$m jusqu'à 2,5 mm.

Les pansements selon l'invention peuvent comporter, sur la face de la couche de masse adhésive hydrocolloïde destinée à être au contact de la peau du patient, un film de protection. Ce dernier peut avantageusement être constitué d'un film siliconé ou d'un papier siliconé. Les films siliconés sont souvent réalisés à partir de polyesters. Les papiers pour papier siliconé sont généralement ici des papiers denses super-calendrés. Il existe également des films complexes à base à la fois de papier et de film plastique.

Le film de protection d'un pansement selon l'invention, afin d'être préhensible, présentera de préférence une épaisseur comprise entre au moins 30 $\mu$m et au plus 300 $\mu$m.

Les pansements selon la présente invention sont fabriqués, de préférence, selon le procédé suivant :

a) on forme un premier élément stratifié en solidarisant entre eux un film support et la masse adhésive hydrocolloïde extrudée ;
b) on met en forme ledit élément stratifié pour obtenir un produit intermédiaire comportant une pluralité de zones formant chacune une ébauche de pansement ;
c) on forme un second élément stratifié en solidarisant entre eux ledit produit intermédiaire et un film de protection ;
d) on découpe ledit second élément stratifié pour former ainsi une pluralité de pansements.

## Exemples

### Exemple comparatif de formulation 1

Une masse adhésive hydrocolloïde contenant deux résines tackifiantes d'hydrocarbure hydrogéné, mais dont les points de ramollissement diffèrent par 50°C, a été préparé avec la composition suivante :

| N° | Composés | Quantité (en poids pour 100 grammes) |
|---|---|---|
| 1 | Elastomère<br>Copolymère bloc Styrène-Isoprène-Styrène/ Styrène Isoprène (commercialisé sous la dénomination KRATON® D 1111 K par KRATON) | 18,0 |
| 2 | Plastifiant<br>Diethylhexyl adipate (commercialisé sous la dénomination Crodamol DOA par Croda) | 7,0 |
| 3 | Résine tackifiante<br>Résine (commercialisé sous la dénomination de Arkon P 140® par Arakawa) | 13,0 |
| 4 | Résine tackifiante<br>Résine (commercialisé sous la dénomination de Arkon P 90® par Arakawa) | 20,0 |
| 5 | Hydrocolloïde<br>Carboxyméthylcellulose (commercialisé sous la dénomination CMC Blanose® 7H4XF par Hercules) | 41,0 |

(suite)

| N° | Composés | Quantité (en poids pour 100 grammes) |
|---|---|---|
| 6 | Antioxvdant<br>Dibutyldithiocarbamate de Zinc (commercialisé sous la dénomination Perkacit® ZDBC par la société FLEXSYS) | 0,5 |
| 7 | Antioxydant<br>Pentaérythritol Tetrakis (3-3,5-di-tert-butyl-4-hydroxyphényl) proprionate) (commercialisé sous la dénomination Irganox® 1010 par Ciba Speciality Chemicals) | 0,5 |

Cette masse adhésive hydrocolloïde a été préparée par la mise en oeuvre du procédé suivant :

Dans un malaxeur à bras en Z à une température de consigne de 135°C, on a introduit les composés 1,2,6 et 7.
A la 45$^{eme}$ minute, on a ajouté le composé 3 et 4.
A la 55$^{ème}$ minute, on a ajouté le composé 5.
La vidange du malaxeur a été effectuée à la 65$^{ème}$ minute.

**Exemple de formulation 1 selon l'invention**

Une masse adhésive hydrocolloïde contenant deux résines tackifiantes d'hydrocarbure hydrogéné, dont les points de ramollissement diffèrent par 35°C, a été préparé avec la composition suivante :

| No | Composés | Quantité (en poids pour 100 grammes) |
|---|---|---|
| 1 | Elastomère<br>Copolymère bloc Styrène-Isoprène-Styrène/ Styrène Isoprène (commercialisé sous la dénomination KRATON® D 1111 K par KRATON) | 18,0 |
| 2 | Plastifiant<br>Diethylhexyl adipate (commercialisé sous la dénomination Crodamol DOA par Croda) | 10,0 |
| 3 | Résine tackifiante<br>Résine (commercialisé sous la dénomination de Arkon P 125® par Arakawa) | 15,0 |
| 4 | Résine tackifiante<br>Résine (commercialisé sous la dénomination de Arkon P 90® par Arakawa) | 15,0 |
| 5 | Hydrocolloïde<br>Carboxyméthylcellulose (commercialisé sous la dénomination CMC Blanose® 7H4XF par Hercules) | 41,0 |
| 6 | Antioxydant<br>Dibutyldithiocarbamate de Zinc (commercialisé sous la dénomination Perkacit® ZDBC par la société FLEXSYS) | 0,5 |
| 7 | Antioxydant<br>Pentaérythritol Tetrakis (3-3,5-di-tert-butyl-4-hydroxyphényl) proprionate) (commercialisé sous la dénomination Irganox® 1010 par Ciba Speciality Chemicals) | 0,5 |

Cette masse adhésive hydrocolloïde a été préparée par la mise en oeuvre du procédé suivant :

Dans un malaxeur à bras en Z à une température de consigne de 135°C, on a introduit les composés 1,2,6 et 7.
A la 45$^{ème}$ minute, on a ajouté le composé 3 et 4.

A la 55ème minute, on a ajouté le composé 5.
La vidange du malaxeur a été effectuée à la 65ème minute.

**Evaluation des propriétés de masses adhésives selon les exemples de formulation**

Evaluation des fluages des masses

Le fluage permet de déterminer le pouvoir adhésif tangentiel d'un matériau adhésivé.

La résistance au cisaillement ou fluage sur une surface standard est définie comme étant le temps nécessaire pour séparer par glissement une surface de matériau enduit d'adhésif sensible à la pression d'une surface standard (lxL) plane dans une direction parallèle à la surface de cette dernière, le tout sous une contrainte de cisaillement initial de :

$$\tau_0 = \frac{M \times g}{l \times L}$$

Les temps de séparation ont été mesurés en laboratoire par un dispositif représenté à la Figure 2.

Le produit à tester est conditionné pendant 24 heures à 21±3°C et à l'humidité relative de 60 ±15%.

L'épaisseur de la masse adhésive hydrocolloïde testée est de 1 mm. Le support utilisé dans les résultats présentés est un film de polyester de 23 µm d'épaisseur enduit d'une masse hot melt constitué de 80% de polymère à base d'ester acrylique (commercialisé par BASF sous la dénomination commerciale acResin A 203 UV) et de 20% d'esters de colophane partiellement hydrogénée (commercialisé par Eastman sous la dénomination commerciale Foral® 85-E Esters de colophane partiellement hydrogénée). La masse hot melt est préparée dans un malaxeur dont la température de consigne est de 100°C. A T0 on introduit les deux composés dans le malaxeur dont la vitesse de rotation des bras est de 20 tours/min. A T=20 minutes, on effectue un grattage des parois du malaxeur (afin de bien incorporer l'ensemble des composés au mélange), la vitesse de rotation des bras est augmentée à 40 tours/min. La température du mélange atteint progressivement 80°C. Après 45 minutes dans le malaxeur, on effectue la vidange du malaxeur, la vitesse de rotation des bras est alors diminuée à 10 tours/min. Le polyester est alors enduit de cette masse. La vitesse de déroulement du polyester est de 5 m/min, la quantité de masse enduite est de 40±3 g/m$^2$. La masse est alors réticulée par un rayonnement ultra-violet d'une puissance de 50 mJ.

Le protecteur utilisé lors de la préparation des échantillons, et ensuite enlevé, est un papier siliconé de 55 µm d'épaisseur.

Les plaques des tests (en acier) sont nettoyées à l'éthanol.

L'échantillon est préparé et accosté à 21±3°C et à l'humidité relative 60 ±15%.

Le protecteur du pansement est enlevé.

Un échantillon de dimensions 25 mm*100 mm est découpé.

Une longueur de 25 mm de l'échantillon à tester est posée sur la plaque de test (en acier) en ayant soin de n'inclure aucune bulle d'air et d'assurer un contact intime.

Le reste de l'échantillon (75 mm) est utilisé pour attacher un triangle de fixation à l'aide d'une pince de fixation.

2 aller retour sont appliqués avec un rouleau métallique à la vitesse de 60 cm/min avec une pression d'accostage de 2kg/cm, soit 5 kg dans le cas présent.

On laisse conditionner pendant 10 min.

Une ligne est marquée sur la plaque à la limite supérieure de la bande testée. Des plaques pré-gravées peuvent être utilisées.

La plaque est suspendue sur le dispositif de fluage dans une enceinte thermorégulée à la température de 40° C et une humidité relative inférieure à 20% avec un angle $\alpha$ = 2°.

Le compteur horaire est alors initialisé.

La masse M de 1 kg est attachée au triangle de fixation (celle-ci déclenche automatiquement le compteur horaire).

On attend afin d'évaluer le glissement (distance de fluage d) et cela jusqu'à ce que les échantillons se décrochent de la plaque. A l'aide de cette méthode, on mesure le temps de rupture. Plus le temps de fluage est élevé, plus la masse est dure. Au contraire, lorsque le temps de fluage est bas, la masse se déforme plus facilement.

Les performances des masses adhésives formulations décrites plus haut ont été comparées dans ce test entre elles ainsi qu'avec celle de la masse adhésive commerciale Compeed®, et les résultats sont présentés dans le tableau suivant :

| Formule | Temps de rupture (min) | Ecarts types | Type de rupture |
|---|---|---|---|
| Compeed® | 50 | 5,5 | Rupture de cohésion |
| Exemple 1 | 27 | 4,4 | Rupture de cohésion |
| Exemple comparatif 1 | >3h | 3,5 | Rupture adhésive |

Dans ce même test, si le temps de tenue est suffisamment long et si le profil de rupture est cohésif, on peut relier le temps de tenue t à la viscosité par la relation :

$$t = L^2 l\eta / 2eMg$$

dans laquelle η) est la viscosité et e l'épaisseur du joint adhésif.

De ces résultats, il a été donc possible de déterminer des viscosités :

| Formule | Viscosité (MPa.s) | Ecarts types |
|---|---|---|
| Compeed® | 3,8 | 0,41 |
| Exemple 1 | 2,0 | 0,33 |
| Exemple comparatif 1 | > 13,5 | na |

De façon générale, plus le temps de fluage est élevé et plus la masse est dure. La masse selon l'Exemple 1 est celle qui se déforme le plus et par conséquent qui à une meilleure complaisance (capacité à épouser les aspérités de la surface sur laquelle elle est appliquée). On peut imaginer qu'une telle masse pénètre donc plus facilement les plis de la peau et que ceci aurait pour effet d'augmenter la surface d'adhésion de la masse et par conséquent d'améliorer sa tenue dans le temps.

De par les résultats observés, la Demanderesse considère que la masse adhésive hydrocolloïde selon l'invention devrait présenter de préférence une viscosité, mesurée selon le protocole indiqué plus haut, entre au moins 1,5 et au plus 4,0 MPa.s, de préférence 1,5 à 3,5 MPa.s.

Résultats sur porteurs

Des pansements comprenant la masse adhésive hydrocolloïde selon l'Exemple 1 ont été testés par des porteurs volontaires pratiquant des activités sportives. Il en est ressorti que les pansements selon l'invention présentent un taux réduit de décollement, une certaine résistance à l'eau, confère une sensation de protection de la peau (protection contre les pressions, les frottements de la chaussure) et ne transfèrent pas de quantité significative de massé adhésive sur la peau.

Dans une étude, des ailettes de protection d'une masse adhésive de pansement ampoule de forme ovale de la marque Compeed® ont été retirées et remplacées par des ailettes neutres. Un pansement ampoule également de forme ovale et de la même taille et épaisseur a été préparé avec la masse adhésive selon l'exemple de formulation 1 de la présente invention. Trente-deux personnes ont participé à cette première étude qui a débuté par 1h ou 1h30 de course à pied puis s'est poursuivi sur 48h en condition urbaine avec cependant une activité sportive renouvelée (marche ou course) pour 50% des personnes.

Dès la fin de la course, on observe une différence significative entre les deux pansements au niveau de l'état de décollement : les pansements selon l'invention se décollent moins que les pansements Compeed® et cela se confirme après 48h de port. En effet, 63% des pansements selon l'invention sont encore en place après 48h de port et sont peu décollés alors que 47% des pansements Compeed® sont présents avec un état de décollement variable.

Les résultats de cette première étude sont présentés à la Figure 3.

D'autres tests semblables avec des porteurs sportifs volontaires, comparant des formes haricot et ovale avec la même masse adhésive hydrocolloïde selon l'exemple de formulation selon l'invention, ont permis de démontrer que la forme n'a pas d'impact sur la tenue du pansement (profil de perte dans le temps de pansement).

**Mise en évidence des propriétés du pansement après vieillissement**

**Exemple comparatif de formulation 2**

On a préparé une masse adhésive hydrocolloïde constituée des composés suivants (quantité exprimée en poids pour 100 grammes de masse) :

| No | Composés | Quantité (en poids pour 100 grammes) |
|---|---|---|
| 1 | Elastomère<br>Copolymère bloc Styrène-Isoprène-Styrène/ Styrène Isoprène (commercialisé sous la dénomination KRATON® D 1111 K par KRATON) | 18,0 |
| 2 | Plastifiant<br>Diethylhexyl adipate (commercialisé sous la dénomination Crodamol DOA par Croda) | 10,0 |
| 3 | Résine tackifiante<br>Résine (commercialisé sous la dénomination de Arkon P 125® par Arakawa) | 30,0 |
| 4 | Hydrocolloïde<br>Carboxyméthylcellulose (commercialisé sous la dénomination CMC Blanose® 7H4XF par Hercules) | 41,0 |
| 5 | Antioxydant<br>Dibutyldithiocarbamate de Zinc (commercialisé sous la dénomination Perkacit® ZDBC par la société FLEXSYS) | 0,5 |
| 6 | Antioxydant<br>Pentaérythritol Tetrakis (3-3,5-di-tert-butyl-4-hydroxyphényl) proprionate) (commercialisé sous la dénomination Irganox® 1010 par Ciba Speciality Chemicals) | 0,5 |

Cette masse adhésive hydrocolloïde a été préparée par la mise en oeuvre du procédé suivant :

Dans un malaxeur à bras en Z à une température de consigne de 135°C, on a introduit les composés 1, 2, 5 et 6.
A la $45^{eme}$ minute, on a ajouté le composé 3.
A la $55^{eme}$ minute, on a ajouté le composé 4.
La vidange du malaxeur a été effectuée à la $65^{eme}$ minute.

**Etude du vieillissement des pansements**

On a mesuré l'adhésivité des pansements tel que préparés à l'exemple comparatif de formulation 2 et l'exemple de formulation 1 selon l'invention, afin d'évaluer la différence de comportement après un vieillissement de 18 mois et 24 mois dans une étuve à 21 $\pm$2°C à une humidité relative de 60 $\pm$15 % HR.
A cet effet, on a utilisé la méthode dite du "tack à la sonde" qui vise à mesurer le décollement d'une sonde cylindrique appliquée sur un adhésif avec une contrainte de pression $P_0$ pendant un temps t donné dans des conditions de température et d'humidité prédéterminées.

Appareillage

Plus précisément, cette méthode a été mise en oeuvre au moyen de l'appareil représenté à la figure 4.
Cet appareil est essentiellement constitué :

- d'une sonde cylindrique 1 présentant une extrémité finement polie susceptible de se déplacer longitudinalement (de bas en haut dans l'exemple représenté) au travers d'un guide sonde 2 constitué d'un cylindre percé en son centre d'un trou libre permettant le libre passage sans frottement de la sonde ;

- d'un dynamomètre électronique comprenant une mâchoire mobile 4 pouvant être reliée à la sonde 1 par une chaînette 5 et un socle 6 sur lequel peut être emboîté un plateau 7 ; ledit dynamomètre étant en outre relié à un système d'acquisition et d'enregistrement des données telles qu'en particulier la vitesse de descente de la sonde, la pression d'accostage de la sonde sur l'adhésif, le temps de contact entre la sonde et l'adhésif et la vitesse de remontée de la sonde.

Echantillons

Masse enduite sur un film de polyuréthane PU inspire 2304 EXOPACK de 30 $\mu$m d'épaisseur.
Les échantillons découpés ont une surface supérieure à la base du guide sonde.

Mode opératoire :

- On a emboîté le plateau 7 sur le socle 6 du dynamomètre.
- On a fixé la chaînette 5 de la sonde dans la mâchoire mobile 4 du dynamomètre.
- On a nettoyé la sonde 1 avec un solvant (cette opération étant répétée avant chaque mesure).
- On a appliqué l'échantillon face à tester sur la base du guide-sonde.
- On a programmé les paramètres du dynamomètre (vitesse de descente, pression d'accostage, temps de contact et vitesse de remontée).
- On a procédé à la mesure en effectuant les opérations suivantes: descente de la sonde
- On a enregistré la force de décollement en kPa que l'on a traduite en contrainte d'arrachement C suivant la relation :

$$C = F/S$$

où F = Force exprimée en N et S= Surface exprimée en $m^2$.

Paramètres du test:

Contrainte d'application : 4.3 kPa (sonde en laiton de 35 g et diamètre de 10 mm)
Vitesse d'essai : 300 mm/min
Temps de contact : 5 s

Les mesures ont été réalisées sur 10 échantillons de chacune des masses.

Résultats :

Les moyennes des résultats des mesures ainsi effectuées exprimés en kPa ont été reportées dans le tableau I.
Le comportement en vieillissement a été évalué de manière comparative entre la masse de l'exemple comparatif de formulation 1 et l'exemple de formulation 1 selon l'invention sur deux configurations de protecteurs. Silphan est un film protecteur en polyester PET siliconé et R1010 est un papier super calandré siliconé.
L'analyse faite avec deux protecteurs a pour but de montrer qu'une des deux masses présente réellement un comportement en vieillissement significativement plus faible que l'autre et ce indépendamment de l'état de surface induit par les protecteurs.
Les données issues des mesures de tack à la sonde montrent un comportement en vieillissement très différent entre la masse de l'exemple comparatif de formulation 2 et l'exemple de formulation 1 selon l'invention. Le tableau montre que le niveau de tack est constant sur la masse de l'exemple 1 selon l'invention et ce pour les deux protecteurs. A l'inverse, sans utiliser de tests statistiques, les données montrent que la masse de l'exemple comparatif de formulation 2 est significativement moins stable que la masse de l'exemple 1 selon l'invention et ce pour les deux protecteurs car le niveau de tack à la sonde décroît avec le temps.

Tableau I

| | | | |
|---|---|---|---|
| Tack à la sonde (10 mm) en kPa à T0 (date de fabrication) | Exemple 1 selon l'nvention | Protecteur Silphan | 104 |
| | | Protecteur R1010 | 67 |
| | Exemple comparatif 2 | Protecteur Silphan | 139 |
| | | Protecteur R1010 | 98 |
| Tack à la sonde (10 mm) en kPa après 18 mois de vieillissement à 25°C | Exemple 1 selon l'invention | Protecteur Silphan | 94 |
| | | Protecteur R1010 | 53 |
| | Exemple comparatif 2 | Protecteur Silphan | 121 |
| | | Protecteu R1010 | 86 |
| Tack à la sonde (10 mm) en kPa après 24 mois de vieilliissement à 25°C | Exemple 1 selon l'invention | Protecteur Silphan | 102 |
| | | Protecteur R1010 | 70 |
| | Exemple comparatif 2 | Protecteur Silphan | 108 |
| | | Protecteur R1010 | 67 |

**Revendications**

**1.** Pansement comprenant une masse adhésive hydrocolloïde comprenant :

- une matrice élastomérique hydrophobe comprenant des copolymères séquencés poly(styrène-oléfine-styrène),
- des particules d'hydrocolloïde(s) dispersées dans ladite matrice élastomérique,
- au moins deux résines tackifiantes d'hydrocarbure hydrogéné, la différence entre les températures de ramollissement d'une première résine tackifiante d'hydrocarbure hydrogéné et d'une deuxième résine tackifiante d'hydrocarbure hydrogéné comprises dans la masse adhésive hydrocolloïde étant d'au moins 10°C et d'au plus 40°C.

**2.** Pansement selon la revendication 1, dans lequel la différence entre les températures de ramollissement d'une première résine tackifiante d'hydrocarbure hydrogéné et d'une deuxième résine tackifiante d'hydrocarbure hydrogéné comprises dans la masse adhésive hydrocolloïde est d'au moins 20°C et d'au plus 40°C, de préférence d'au moins 30°C et d'au plus 40°C.

**3.** Pansement selon la revendication 1 ou 2, dans lequel lesdits copolymères séquencés poly(styrène-oléfine-styrène) comprennent des copolymères triblocs poly(styrène-isoprène-styrène) et/ou des copolymères diblocs poly(styrène-isoprène).

**4.** Pansement selon l'une quelconque des revendications 1 à 3, dans lequel lesdites particules d'hydrocolloïde(s) comprennent au moins une espèce choisie dans le groupe constitué par : la pectine, les alginates, les gommes végétales naturelles telles que la gomme de Karaya, les dérivés de cellulose tels que les carboxyméthylcelluloses et leurs sels de métal alcalin, notamment de sodium ou de calcium, ainsi que les polymères synthétiques à base de sels de l'acide acrylique.

**5.** Pansement selon l'une quelconque des revendications 1 à 4, dans lequel, par rapport au poids total de la masse adhésive des pansements :

- les élastomères formant la matrice élastomérique sont présents en une quantité d'au moins 10 % et au plus 30 % en poids,
- le(s) hydrocolloïde(s) est (sont) présent(s) en une quantité totale d'hydrocolloïde(s) d'au moins 2 % et-au plus 50 % en poids,
- les résines tackifiantes sont présentes en une quantité d'au moins 10 % et au plus 40 % en poids.

**6.** Pansement selon l'une quelconque des revendications 1 à 5 comprenant en outre un ou plusieurs des agents suivants : antioxydant(s), plastifiant(s), tensioactif(s).

**7.** Pansement selon l'une quelconque des revendications 1 à 6, dans lequel, sur la face de la masse adhésive hydrocolloïde non destinée à être au contact de la peau du patient, le pansement comporte une couche support.

**8.** Pansement selon l'une quelconque des revendications 1 à 7, dans lequel, sur la face de la masse adhésive hydrocolloïde destinée à être au contact de la peau du patient, le pansement comporte un film de protection.

**9.** Pansement selon l'une quelconque des revendications 1 à 8 pour son utilisation dans une méthode de traitement de plaies telles que les plaies exudatives, les brûlures, les lésions dermo-épidermiques superficielles, profondes, chroniques ou aiguës, notamment dans une méthode de traitement de l'ampoule.

**Patentansprüche**

**1.** Verband mit einer haftenden Hydrokolloidmasse, umfassend:

- eine hydrophobe Elastomermatrix, die Poly(styrol-Olefin-Styrol)-Blockcopolymere umfasst,
- Hydrokolloidpartikel, die in der Elastomermatrix verteilt sind,
- wenigstens zwei hydrierte Kohlenwasserstoff-Tackifier-Harze, wobei der Unterschied zwischen den Erweichungstemperaturen eines ersten hydrierten Kohlenwasserstoff-Tackifier-Harzes und eines zweiten hydrierten Kohlenwasserstoff-Tackifier-Harzes, die in der haftenden Hydrokolloidmasse enthalten sind, wenigstens 10°C und höchstens 40 °C beträgt.

**2.** Verband nach Anspruch 1, wobei der Unterschied zwischen den Erweichungstemperaturen eines ersten hydrierten Kohlenwasserstoff-Tackifier-Harzes und eines zweiten hydrierten Kohlenwasserstoff-Tackifier-Harzes, die in der haftenden Hydrokolloidmasse enthalten sind, wenigstens 20 °C und höchstens 40 °C, vorzugsweise wenigstens 30 °C und höchstens 40 °C beträgt.

**3.** Verband nach Anspruch 1 oder 2, wobei die Poly(styrol-Olefin-Styrol)-Blockcopolymere Poly(styrol-Isopren-Styrol)-Dreiblockcopolymere und/oder Poly(styrol-Isopren)-Zweiblockcopolymere umfassen.

**4.** Verband nach einem der Ansprüche 1 bis 3, wobei die Hydrokolloidpartikel wenigstens eine Art umfassen, die aus der Gruppe bestehend aus Pektin, Alginaten, natürlichen Pflanzengummis wie Karaya-Gummi, Cellulosederivaten wie Carboxymethylcellulose und ihre Alkalimetallsalze, insbesondere von Natrium oder Kalzium, sowie synthetischen Polymeren auf der Basis von Salzen der Acrylsäure ausgewählt ist.

**5.** Verband nach einem der Ansprüche 1 bis 4, wobei bezogen auf das Gesamtgewicht der haftenden Masse der Verbände:

- die die Elastomermatrix bildenden Elastomere in einer Menge von wenigstens 10 und höchstens 30 Gew.-% vorliegen,
- das/die Hydrokolloid(e) in einer Gesamtmenge an Hydrokolloid(en) von wenigstens 2 und höchstens 50 Gew.-% vorliegt (vorliegen),
- die Tackifier-Harze in einer Menge von wenigstens 10 und höchstens 40 Gew.-% vorliegen.

**6.** Verband nach einem der Ansprüche 1 bis 5, ferner umfassend eines oder mehrere der folgenden Mittel: Antioxidationsmittel, Weichmacher, Tensid(e).

**7.** Verband nach einem der Ansprüche 1 bis 6, wobei auf der Seite der haftenden Hydrokolloidmasse, die nicht dazu bestimmt ist, mit der Haut des Patienten in Kontakt zu sein, der Verband eine Tragschicht umfasst.

**8.** Verband nach einem der Ansprüche 1 bis 7, wobei auf der Seite der haftenden Hydrokolloidmasse, die dazu bestimmt ist, mit der Haut des Patienten in Kontakt zu sein, der Verband einen Schutzfilm umfasst.

**9.** Verband nach einem der Ansprüche 1 bis 8, für seine Verwendung bei einem Verfahren zur Behandlung von Wunden, wie exsudativen Wunden, Verbrennungen, oberflächlichen, tiefen, chronischen oder akuten dermoepidermalen Verletzungen, insbesondere bei einem Verfahren zur Blasenbehandlung.

## Claims

1. A wound dressing which comprises a hydrocolloid adhesive mass comprising:

   - a hydrophobic elastomer matrix comprising poly(styrene-olefin-styrene) block copolymers,
   - particles of hydrocolloid(s) dispersed in said elastomer matrix,
   - at least two hydrogenated-hydrocarbon tackifying resins, the difference between the softening temperatures of a first hydrogenated-hydrocarbon tackifying resin and of a second hydrogenated-hydrocarbon tackifying resin comprised in the hydrocolloid adhesive mass being at least 10°C and at most 40°C.

2. The dressing as claimed in claim 1, in which the difference between the softening temperatures of a first hydrogenated-hydrocarbon tackifying resin and of a second hydrogenated-hydrocarbon tackifying resin comprised in the hydrocolloid adhesive mass is at least 20°C and at most 40°C, preferably at least 30°C and at most 40°C.

3. The dressing as claimed in claim 1 or 2, in which said poly(styrene-olefin-styrene) block copolymers comprise poly(styrene-isoprene-styrene) triblock copolymers and/or poly(styrene-isoprene) diblock copolymers.

4. The dressing as claimed in claim 1 to 3, in which said particles of hydrocolloid(s) comprise at least one entity chosen from the group consisting of: pectin, alginates, natural plant gums such as karaya gum, cellulose derivatives such as carboxymethylcelluloses and their alkali metal, in particular sodium or calcium, salts, and also synthetic polymers based on acrylic acid salts.

5. The dressing as claimed in any one of claims 1 to 4, in which, relative to the total weight of the adhesive mass of the dressings:

   - the elastomers forming the elastomer matrix are present in an amount of at least 10% and at most 30% by weight,
   - the hydrocolloid(s) is (are) present in a total amount of hydrocolloid(s) of at least 2% and at most 50% by weight,
   - the tackifying resins are present in an amount of at least 10% and at most 40% by weight.

6. The dressing as claimed in any one of claims 1 to 5, also comprising one or more of the following agents: antioxidant(s), plasticizer(s) and surfactant(s).

7. The dressing as claimed in any one of claims 1 to 6, in which the dressing comprises a backing layer on the face of the hydrocolloid adhesive mass not intended to be in contact with the patient's skin.

8. The dressing as claimed in any one of claims 1 to 7, in which the dressing comprises a protective film on the face of the hydrocolloid adhesive mass intended to be in contact with the patient's skin.

9. The dressing as claimed in any one of claims 1 to 8, for use thereof in a method for treating wounds, such as exudative wounds, burns, superficial or deep dermo-epidermal lesions, which may be chronic or acute, in particular in a method for treating blisters.

Figure 1

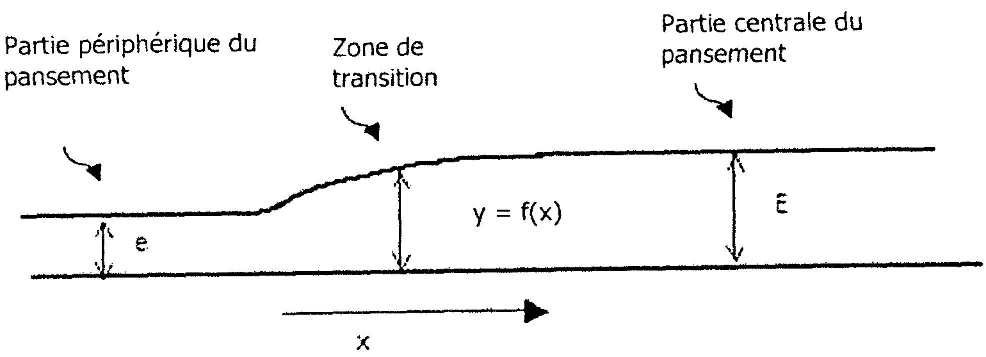
Partie périphérique du pansement
Zone de transition
Partie centrale du pansement
e
y = f(x)
E
x

Figure 2

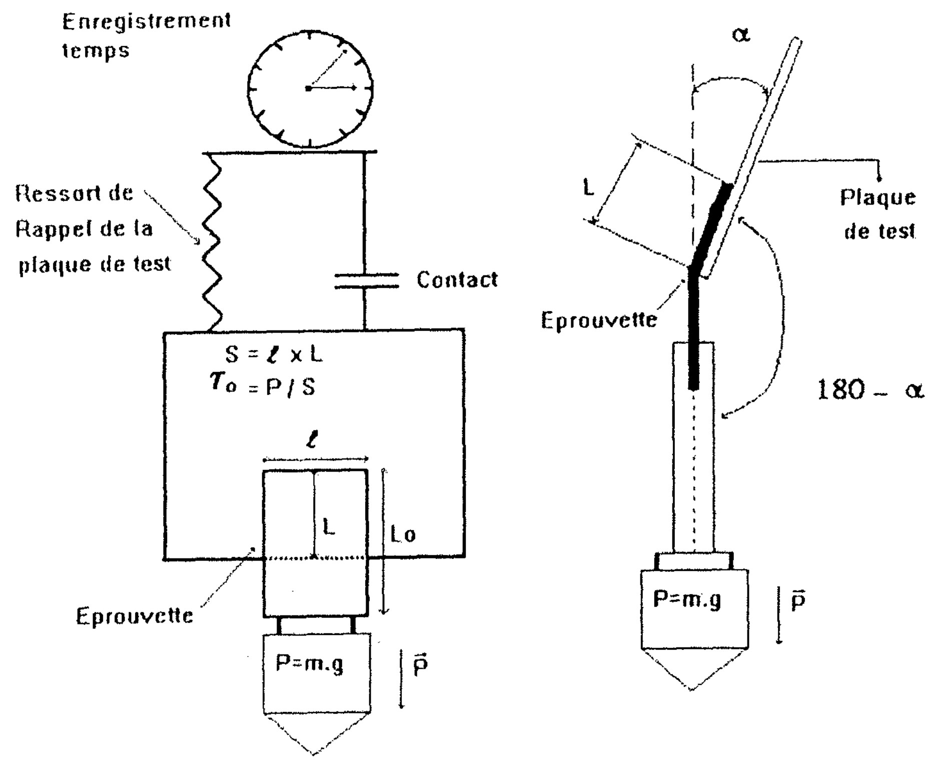
Enregistrement
temps
Ressort de
Rappel de la
plaque de test
Contact
S = ℓ x L
To = P / S
ℓ
L
Lo
Eprouvette
P=m.g
P
α
L
Plaque
de test
Eprouvette
180 - α
P=m.g
P

Figure 3

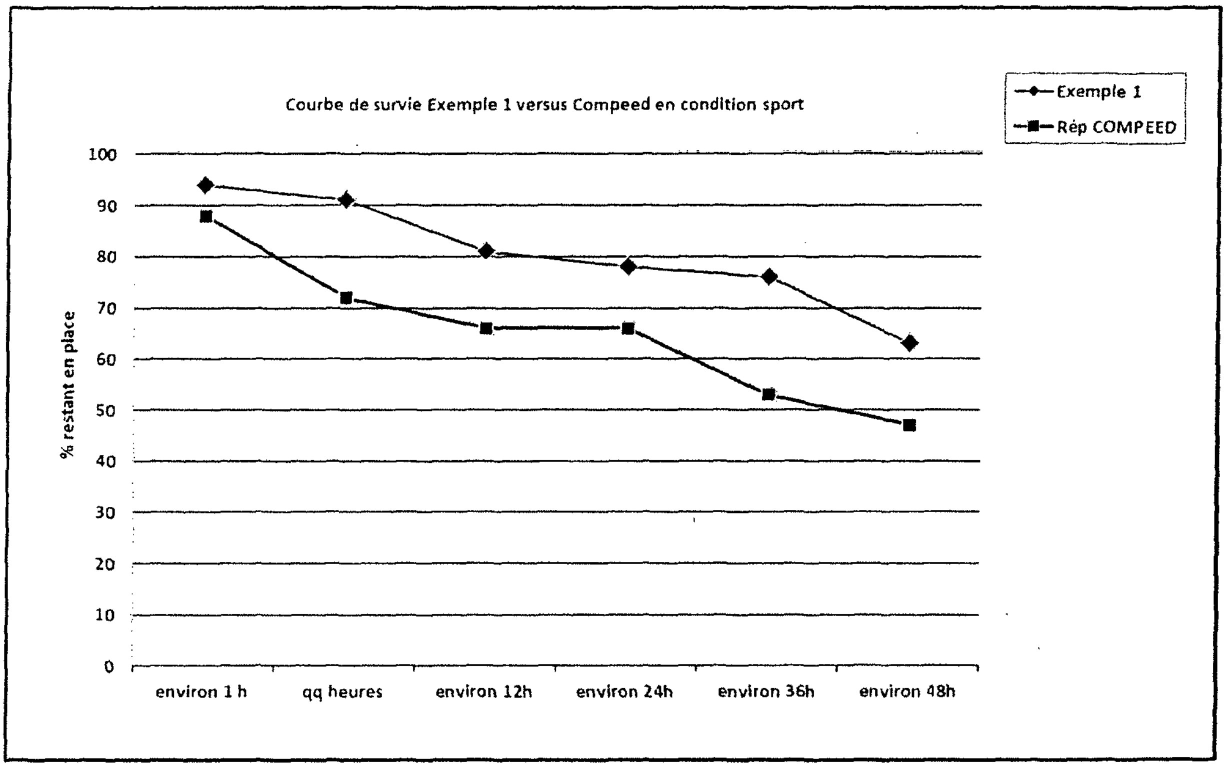
Courbe de survie Exemple 1 versus Compeed en condition sport
Exemple 1
Rép COMPEED
% restant en place
100
90
80
70
60
50
40
30
20
10
0
environ 1 h
qq heures
environ 12h
environ 24h
environ 36h
environ 48h

Figure 4

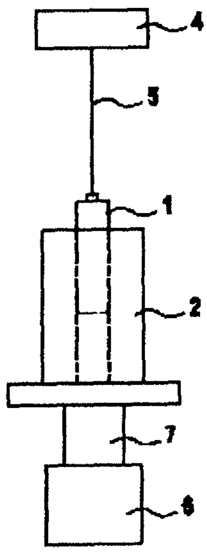

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2495473
- FR 2775903
- EP 0927051 A
- EP 1165717 A
- EP 0264299 A
- WO 9205755 A
- EP 1020198 A
- WO 2010004222 A